(19) **Europäisches Patentamt / European Patent Office / Office européen des brevets**

(11) **EP 3 115 058 A1**

(12) **DEMANDE DE BREVET EUROPEEN**

(43) Date de publication:
**11.01.2017 Bulletin 2017/02**

(51) Int Cl.:
**A61K 39/00** (2006.01)

(21) Numéro de dépôt: **15176174.9**

(22) Date de dépôt: **09.07.2015**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**MA**

(71) Demandeur: **Institut Gustave Roussy**
**94800 Villejuif (FR)**

(72) Inventeurs:
- **MAMI-CHOUAIB, Fathia**
  **92340 Bourg la Reine (FR)**
- **DURGEAU, Aurélie**
  **91400 Orsay (FR)**

(74) Mandataire: **Gevers & Orès**
**41 avenue de Friedland**
**75008 Paris (FR)**

(54) **PEPTIDES IMMUNOGENES DE PREPROCALCITONINE**

(57) L'invention est relative à des combinaisons de peptides portant des épitopes T de l'antigène préprocalcitonine, présentés par le CMH.
Ces peptides sont utilisables en immunothérapie anti-tumorale.

EP 3 115 058 A1

SEQUENCE LISTING

<110> INSTITUT GUSTAVE ROUSSY
Fathia, MAMI-CHOUAIB
Aurélie, DURGEAU

<120> PEPTIDES IMMUNOGENES DE PREPROCALCITONINE

<130> VMA/sf - F1534/21/EP

<160> 18

<170> PatentIn version 3.5

<210> 1
<211> 15
<212> PRT
<213> Artificial Sequence

<220>
<223> ppCT1-15

<400> 1

Met Gly Phe Gln Lys Phe Ser Pro Phe Leu Ala Leu Ser Ile Leu
1               5                   10                  15

<210> 2
<211> 15
<212> PRT
<213> Artificial Sequence

<220>
<223> ppCT71-85

<400> 2

Glu Arg Glu Gly Ser Ser Leu Asp Ser Pro Arg Ser Lys Arg Cys
1               5                   10                  15

<210> 3
<211> 15
<212> PRT
<213> Artificial Sequence

<220>
<223> ppCT86-100

<400> 3

Gly Asn Leu Ser Thr Cys Met Leu Gly Thr Tyr Thr Gln Asp Phe
1               5                   10                  15

<210> 4
<211> 9
<212> PRT
<213> Artificial Sequence

<220>
<223> ppCT9-17

<400> 4

Phe Leu Ala Leu Ser Ile Leu Val Leu
1               5


<210> 5
<211> 10
<212> PRT
<213> Artificial Sequence

<220>
<223> ppCT16-25

<400> 5

Val Leu Leu Gln Ala Gly Ser Leu His Ala
1               5               10


<210> 6
<211> 10
<212> PRT
<213> Artificial Sequence

<220>
<223> ppCT50-59

<400> 6

Leu Leu Ala Ala Leu Val Gln Asp Tyr Val
1               5               10


<210> 7
<211> 10
<212> PRT
<213> Artificial Sequence

<220>
<223> ppCT91-100

<400> 7

Cys Met Leu Gly Thr Tyr Thr Gln Asp Phe
1               5               10


<210> 8
<211> 15
<212> PRT
<213> Artificial Sequence

<220>
<223> ppCT45-60

<400> 8

Glu Ala Arg Leu Leu Leu Ala Ala Leu Val Gln Asp Tyr Val Gln
1               5               10              15

```
<210>  9
<211>  10
<212>  PRT
<213>  Artificial Sequence

<220>
<223>  ppCT1-10

<400>  9

Met Gly Phe Gln Lys Phe Ser Pro Phe Leu
1               5                   10


<210>  10
<211>  9
<212>  PRT
<213>  Artificial Sequence

<220>
<223>  ppCT3-12

<400>  10

Phe Gln Lys Phe Pro Phe Leu Ala Leu
1               5


<210>  11
<211>  10
<212>  PRT
<213>  Artificial Sequence

<220>
<223>  ppCT5-14

<400>  11

Lys Phe Ser Pro Phe Leu Ala Leu Ser Ile
1               5                   10


<210>  12
<211>  10
<212>  PRT
<213>  Artificial Sequence

<220>
<223>  ppCT6-15

<400>  12

Phe Ser Pro Phe Leu Ala Leu Ser Ile Leu
1               5                   10


<210>  13
<211>  10
<212>  PRT
<213>  Artificial Sequence

<220>
<223>  ppCT87-96
```

<400> 13

Asn Leu Ser Thr Cys Met Leu Gly Thr Tyr
1               5               10

<210> 14
<211> 10
<212> PRT
<213> Artificial Sequence

<220>
<223> ppCT88-97

<400> 14

Leu Ser Thr Cys Met Leu Gly Thr Tyr Thr
1               5               10

<210> 15
<211> 10
<212> PRT
<213> Artificial Sequence

<220>
<223> ppCT96-105

<400> 15

Tyr Thr Gln Asp Phe Asn Lys Phe His Thr
1               5               10

<210> 16
<211> 10
<212> PRT
<213> Artificial Sequence

<220>
<223> ppCT41-50

<400> 16

Thr Leu Ser Glu Asp Glu Ala Arg Leu Leu
1               5               10

<210> 17
<211> 10
<212> PRT
<213> Artificial Sequence

<220>
<223> ppCT53-62; et

<400> 17

Ala Leu Val Gln Asp Tyr Val Gln Met Lys
1               5               10

```
<210>  18
<211>  9
<212>  PRT
<213>  Artificial Sequence

<220>
<223>  ppCT58-66

<400>  18

Tyr Val Gln Met Lys Ala Ser Glu Leu
1               5
```

## Description

La présente invention est relative à des combinaisons d'épitopes de la préprocalcitonine (ppCT) et à leur utilisation en immunothérapie antitumorale.

La vaccination ou immunothérapie peptidique est une approche thérapeutique qui fait actuellement l'objet d'un grand intérêt dans le cadre de la prévention ou du traitement des cancers. Son principe repose sur l'immunisation par des peptides reproduisant des épitopes T d'antigènes tumoraux reconnus par les lymphocytes T cytotoxiques (CTL), qui jouent un rôle majeur dans l'élimination des cellules cancéreuses exprimant ces antigènes à leur surface.

On rappellera que les CTL ne reconnaissent pas les antigènes protéiques entiers, mais des fragments peptidiques de ceux-ci, présentés par les molécules du complexe majeur d'histocompatibilité (CMH) exprimées à la surface de différentes cellules. Ce sont ces fragments peptidiques qui constituent les épitopes T.

La présentation de ces peptides résulte d'un processus complexe, dénommé « apprêtement de l'antigène », qui implique 3 étapes principales :

- la dégradation cytosolique des antigènes par un complexe multienzymatique dénommé protéasome ;
- la translocation des peptides issus de cette dégradation dans le réticulum endoplasmique (RE) par les transporteurs TAP ;
- l'association de ces peptides avec le CMH pour former des complexes stables peptide/CMH, qui seront exportés à la surface cellulaire.

Les épitopes présentés par le complexe majeur d'histocompatibilité de classe I (CMH I) ont généralement 8 à 11 acides aminés (aa) et sont reconnus par les cellules T CD8+, qui représentent la composante majeure de la réponse cytotoxique. Les épitopes présentés par le complexe majeur d'histocompatibilité de classe II (CMH II) ont généralement 13 à 18 acides aminés et sont reconnus par les cellules T CD4+.

L'identification de ces épitopes constitue une étape essentielle pour le développement de compositions d'immunothérapie anti-tumorale.

La préprocalcitonine est codée par le gène *CALCA* qui code également pour la forme alpha d'un neuropeptide, le « *calcitonin gene-related peptide* » ($\alpha$-CGRP). Ce gène contient 5 introns et 6 exons, et son transcrit primaire est l'objet d'un épissage alternatif tissu-spécifique. La jonction des exons 1, 2, 3 et 4 produit l'ARNm *calcitonine* dans les cellules C de la thyroïde, tandis que celle des exons 1, 2, 3, 5 et 6 produit l'ARNm $\alpha$-CGRP dans les neurones (MORRIS et al., Nature, 308, 746-8, 1984).

L'ARNm *calcitonine* code pour un précurseur de 141 acides aminés, la préprocalcitonine, qui contient une séquence signal N-terminale de 25 résidus, dont le clivage produit la procalcitonine, de 116 aa. La procalcitonine comprend une région N-terminale de 57 aa, suivie de la calcitonine mature, de 32 aa, et d'un peptide C-terminal de 21 aa, la katacalcine (ROSENFELD et al., Nature, 304, 129-35, 1983). L' $\alpha$-CGRP, sous sa forme mature, est un peptide de 37 aa, à effet vasodilatateur, retrouvé dans un grand nombre de tissus (ZAIDI et al., Crit Rev Clin Lab Sci, 28, 109-74, 1990). La séquence signal de la préprocalcitonine se retrouve également dans la préprohormone de l' $\alpha$-CGRP.

Le rôle physiologique de la calcitonine est principalement la protection du squelette pendant les périodes de « stress calcique », telles que la croissance, la grossesse et la lactation. La calcitonine est également produite en quantités importantes par les cellules des carcinomes médullaires de la thyroïde (CMT) et de certains carcinomes pulmonaires (COOMBES et al., Lancet, 1, 1080-3, 1974; MILHAUD et al., Lancet, 1, 462-3, 1974). Des niveaux élevés de calcitonine plasmatique sont des marqueurs diagnostiques et pronostiques dans ces tumeurs.

Il a été proposé d'utiliser des cellules dendritiques pulsées avec la calcitonine mature pour l'immunothérapie du CMT (SCHOTT et al., Cancer Immunol Immunother, 51, 663-8, 2002). Plus récemment, l'équipe des Inventeurs a identifié un peptide antigénique de 10 acides aminés issu du peptide signal de la préprocalcitonine. Ce peptide correspond à un épitope apprêté dans le réticulum endoplasmique, par un mécanisme indépendant du protéasome et des transporteurs TAP (Demande de brevet WO2009010874).

Les Inventeurs ont maintenant identifié d'autres régions de la préprocalcitonine impliquées dans l'induction d'une réponse immune antitumorale, et montré que l'association de différents épitopes permet une meilleure réponse anti-tumorale.

La présente invention a pour objet une composition comprenant au moins deux séquences peptidiques choisies parmi :

- MGFQKFSPFLALSIL (SEQ ID NO: 1), également dénommée ci-après ppCT1-15 ;
- EREGSSLDSPRSKRC (SEQ ID NO: 2), également dénommée ci-après ppCT71-85;
- GNLSTCMLGTYTQDF (SEQ ID NO: 3), également dénommée ci-après ppCT86-100 ;
- FLALSILVL (SEQ ID NO: 4), également dénommée ci-après ppCT9-17;
- VLLQAGSLHA (SEQ ID NO: 5), également dénommée ci-après ppCT16-25;

- LLAALVQDYV (SEQ ID NO: 6), également dénommée ci-après ppCT50-59;
- CMLGTYTQDF (SEQ ID NO: 7), également dénommée ci-après ppCT91-100;
- EARLLLAALVQDYVQ (SEQ ID NO: 8), également dénommée ci-après ppCT45-60;
- MGFQKFSPFL (SEQ ID NO: 9), également dénommée ci-après ppCT1-10;
- FQKFPFLAL (SEQ ID NO: 10), également dénommée ci-après ppCT3-12;
- KFSPFLALSI (SEQ ID NO: 11), également dénommée ci-après ppCT5-14;
- FSPFLALSIL (SEQ ID NO: 12), également dénommée ci-après ppCT6-15;
- NLSTCMLGTY (SEQ ID NO: 13), également dénommée ci-après ppCT87-96;
- LSTCMLGTYT (SEQ ID NO: 14), également dénommée ci-après ppCT88-97;
- YTQDFNKFHT (SEQ ID NO: 15), également dénommée ci-après ppCT96-105;
- TLSEDEARLL (SEQ ID NO: 16), également dénommée ci-après ppCT41-50;
- ALVQDYVQMK (SEQ ID NO: 17), également dénommée ci-après ppCT53-62; et
- YVQMKASEL (SEQ ID NO: 18), également dénommée ci-après ppCT58-66.

Dans une composition selon l'invention, chacune desdites séquences est présente soit sous forme d'un peptide de 8 à 15 acides aminés, soit incluse dans un polypeptide multiépitopique chimérique. On définit ici un polypeptide chimérique comme une succession d'acides aminés qui n'est pas présente dans la nature. Un polypeptide chimérique tel qu'entendu ici peut comprendre au moins deux séquences choisies parmi les séquences SEQ ID Nos : 1 à 18, lesdites séquences étant, dans ledit polypeptide, adjacentes, liées par un élément de liaison constitué par 1 à 5 acides aminés, ou séparées par une séquence comprenant un épitope d'une protéine autre que la préprocalcitonine.

Les compositions selon l'invention sont donc multiépitopiques, et sont de préférence capables de générer une réponse polyspécifique, au moins chez une partie des individus.

Selon un mode de réalisation préféré de l'invention, la composition comprend au moins une séquence correspondant à un épitope apprêté dans le réticulum endoplasmique par un mécanisme indépendant du protéasome et des transporteurs TAP, telles que les séquences SEQ ID Nos : 4 et 5. De façon encore préférée, la composition comprend également au moins une séquence correspondant à un épitope apprêté dans le réticulum endoplasmique par un mécanisme dépendant du protéasome et des transporteurs TAP, telles que les séquences SEQ ID Nos : 3 et 6. La composition selon l'invention comprend de préférence la séquence SEQ ID No : 6, soit sous forme de peptide constitué de cette séquence, soit incluse dans un polypeptide chimérique tel que défini ci-dessus.

Selon un mode de réalisation particulier de l'invention, la composition comprend un mélange de peptides de 9 à 15 acides aminés. Ce mélange peut être lyophilisé ou en suspension dans une solution adaptée à l'utilisation pharmaceutique.

Selon un autre mode de réalisation préféré de l'invention, la composition comprend un polypeptide chimérique comprenant au moins deux séquences choisies parmi les séquences SEQ ID Nos : 1 à 18, lesdites séquences étant adjacentes, liées par un élément de liaison constitué par 1 à 5 acides aminés, ou séparées par une séquence comprenant un épitope d'une protéine autre que la préprocalcitonine. Le cas échéant, le polypeptide chimérique peut comprendre plusieurs copies d'une même séquence. Également, dans un polypeptide chimérique conforme à l'invention, au moins une partie des séquences immunogènes peut être insérée dans l'adénylate cyclase de *Bordetella pertussis* (CyA). Comme le récepteur de CyA est le même que celui de CD11b, et comme les cellules dendritiques expriment le récepteur CD11b, une telle construction permet de diriger directement un épitope immunogène vers les cellules dendritiques. (DADAGLIO et al., Int. Immunol, 15, 1423-1430, 2003).

Une composition multiépitopique conforme à l'invention peut comprendre, pour être largement utilisable sur une population dont les individus portent des allèles HLA différents, des épitopes présentés par différentes molécules du CMH.

Un polypeptide chimérique conforme à l'invention peut être facilement obtenu par des méthodes connues en elles-mêmes, et notamment par les techniques classiques de l'ADN recombinant.

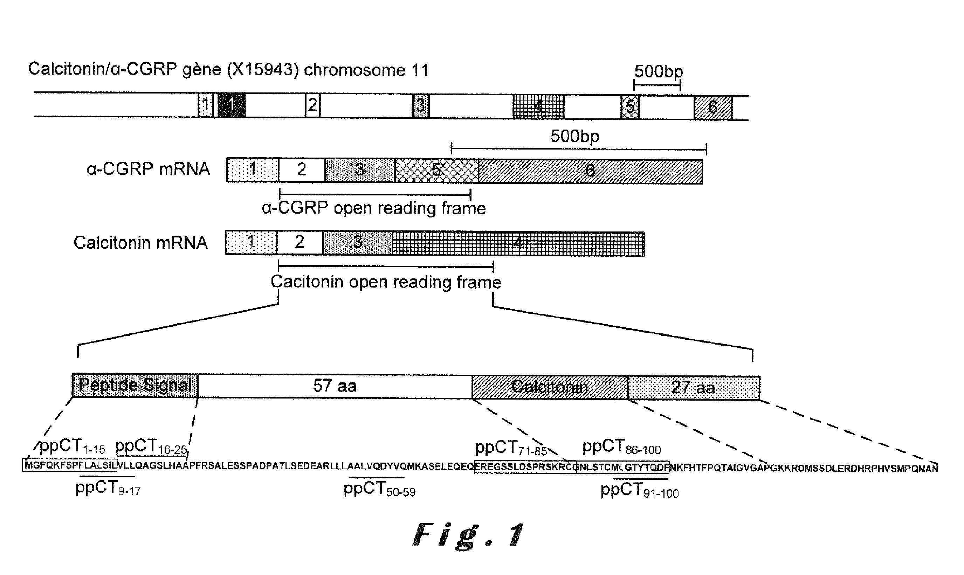

Une composition selon la présente invention comprend de préférence au moins deux séquences peptidiques choisies parmi les séquences SEQ ID Nos : 1 à 7, et de manière encore préférée au moins 3 de ces séquences. La position des peptides SEQ ID NO: 1-7 par rapport à la séquence complète de la préprocalcitonine est représentée sur la Figure 1.

A titre d'exemples non limitatifs de combinaisons de séquences présentes dans une composition selon l'invention, on citera les combinaisons suivantes :

- la combinaison des séquences SEQ ID Nos : 1, 2, 3, 5 et 6 ;
- la combinaison des séquences SEQ ID Nos : 1, 3, 4, 5 et 6 ;
- la combinaison des séquences SEQ ID Nos : 1, 2, 5 et 6 ;
- la combinaison des séquences SEQ ID Nos : 1, 3, 5 et 6 ; et
- la combinaison des séquences SEQ ID Nos : 3, 4, 5 et 6.

La présente invention a également pour objet des molécules d'acide nucléique codant pour un mélange de peptides immunogènes ou pour un polypeptide chimérique tels que définis ci-dessus, ainsi que des compositions les comprenant. Ces polynucléotides peuvent être insérés dans un vecteur d'expression, sous contrôle transcriptionnel d'un promoteur approprié, pour permettre l'expression du peptide immunogène ou du polypeptide chimérique conforme à l'invention dans une cellule ou un organisme hôte. Le choix du vecteur d'expression dépend notamment de la cellule ou de l'organisme (procaryote ou eucaryote) où l'expression est souhaitée. S'il est prévu d'administrer directement le polynucléotide à un patient à traiter, on utilisera de préférence un plasmide d'ADN nu, ou un vecteur permettant une expression transitoire, par exemple un vecteur dérivé d'un adénovirus ou d'un virus de la vaccine. La présente invention porte donc également sur une composition comprenant au moins une molécule d'acide nucléique codant pour au moins deux peptides de 9 à 15 acides aminés choisis parmi les séquences SEQ ID Nos : 1 à 18 ou codant pour un polypeptide chimérique tel décrit plus haut.

D'autres compositions conformes à l'invention peuvent également comprendre des cellules dendritiques, chargées avec un peptide ou une composition multiépitopique de l'invention, ou transformées avec un polynucléotide de l'invention, inséré dans un vecteur d'expression approprié. Elles peuvent également comprendre des cellules présentatrices de l'antigène artificielles chargées avec un peptide ou une composition multiépitopique de l'invention. Ces cellules présentatrices de l'antigène artificielles peuvent être notamment des vésicules dérivées de cellules tumorales (texosomes) décrites dans la Demande PCT WO 1999/003499, ou des exosomes dérivés de cellules dendritiques, comme décrit dans la publication de VIAUD et al. (J Immunother, 34, 65-75, 2011).

Un autre aspect de la présente invention est l'utilisation des compositions décrites plus haut comme médicament, en particulier comme médicament destiné à l'immunothérapie anti-tumorale, plus partculièrement au traitement de tumeurs exprimant la calcitonine et/ou l' $\alpha$-CGRP. Ceci englobe en particulier les carcinomes pulmonaires, à petites cellules ou non, ainsi que les carcinomes médullaires de la thyroïde. Les compositions selon l'invention peuvent également être utilisées pour le traitement de pathologies associées à un taux sérique élevé de calcitonine ou procalcitonine ou pro $\alpha$-CGRP, telles que les pathologies cancéreuses suivantes : cancer du rein, du sein, gastrointestinal (TABOLLI et al, Tumori, 69, 227-230, 1983), du pancréas, de la prostate (SIM et al, Ann Clin Lab Sci., 26, 487-95, 1996), du foie (CONTE et al, Acta Endocrinol, 106, 109-11, 1984) ou des leucémies myélocytaires chroniques (TAKUBO et al Haematologia, 31, 177-9, 2001), des leucémies aiguës indifférenciées et myéloblastiques (KIEFER et al, Leuk Lymphoma., 13, 501-507, 1994), tumeurs neuroendocrines et cancer du foie (GHILLIANI et al, Cancer Res, 49, 6845-6851, 1989).

Une composition selon l'invention peut être utilisée de façon particulièrement avantageuse pour l'immunothérapie d'une tumeur dont les cellules n'expriment pas les transporteurs de peptides TAP.

Les compositions décrites plus haut sont particulièrement appropriées au traitement d'un patient HLA-A*0201.

La présente invention a également pour objet l'utilisation d'une composition multiépitopique d'au moins deux épitopes peptidiques immunogènes, ou d'une molécule d'acide nucléique conforme à l'invention pour l'obtention d'un médicament, et notamment d'un médicament destiné à l'immunothérapie antitumorale, et en particulier au traitement de tumeurs exprimant la calcitonine et/ou l' $\alpha$-CGRP. Ceci englobe en particulier les carcinomes pulmonaires, à petites cellules ou non, ainsi que les carcinomes médullaires de la thyroïde. Ledit médicament peut également être utilisé pour le traitement de pathologies associées à un taux sérique élevé de calcitonine ou précalcitonine ou prépro $\alpha$-CGRP, telles que les pathologies cancéreuses suivantes : cancer du rein, du sein, gastrointestinal (TABOLLI et al, Tumori, 69, 227-230, 1983), du pancréas, de la prostate (SIM et al, Ann Clin Lab Sci., 26, 487-95, 1996), du foie (CONTE et al, Acta Endocrinol, 106, 109-11, 1984) ou des leucémies myélocytaires chroniques (TAKUBO et al Haematologia, 31, 177-9, 2001), des leucémies aiguës indifférenciées et myéloblastiques (KIEFER et al, Leuk Lymphoma., 13, 501-507, 1994), tumeurs neuroendocrines et cancer du foie (GHILLIANI et al, Cancer Res, 49, 6845-6851, 1989).

La présente invention englobe également les médicaments comprenant, en tant que principe actif, au moins un peptide immunogène, une composition, ou une molécule d'acide nucléique conforme à l'invention.

Selon un mode de réalisation préféré de la présente invention, lesdits médicaments sont des vaccins, en particulier des vaccins thérapeutiques.

Des médicaments conformes à l'invention peuvent comprendre en outre les excipients usuels, ainsi que des adjuvants habituellement utilisés en immunothérapie, et permettant par exemple de favoriser l'administration du principe actif, de le stabiliser, d'augmenter son immunogénicité, etc. Des exemples d'adjuvants utilisables incluent les oligodéoxynucléotides CpG, l'Apoptosis-Inducing Factor (AIF), les Heat Shock Proteins (HSP), les récepteurs Toll-like (TLRs) tel que des agonistes de TLR3 (Poly I :C), et des cytokines et chimiokines telles que l'IL-7, l'IL-12, l'IL-15 et le GM-CSF et CCL5 (Rantes).

La présente invention sera mieux comprise à l'aide du complément de description qui va suivre, qui se réfère à des exemples non-limitatifs illustrant l'identification de mélanges de peptides immunogènes conformes à l'invention.

**LISTE DES FIGURES**

Figure 1 : Position des peptides SEQ ID NO: 1-7 par rapport à la séquence complète de la préprocalcitonine.

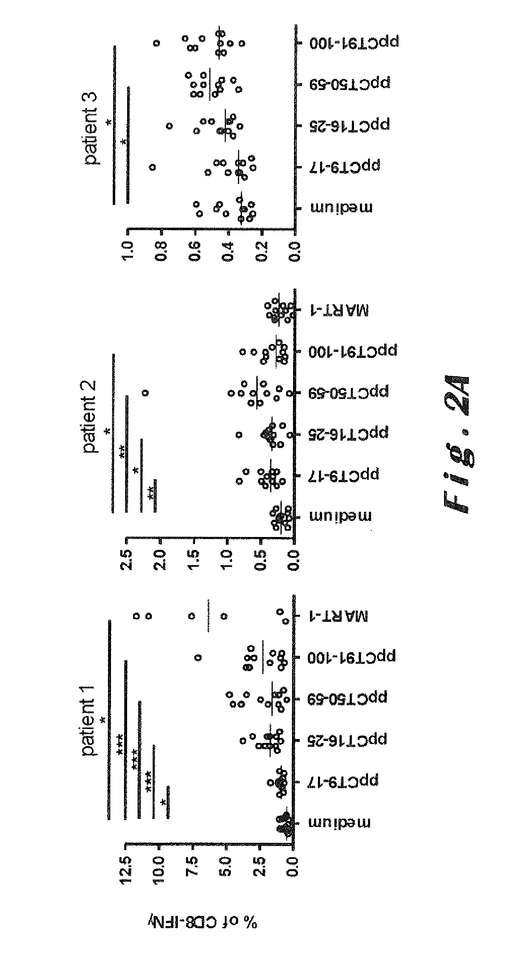

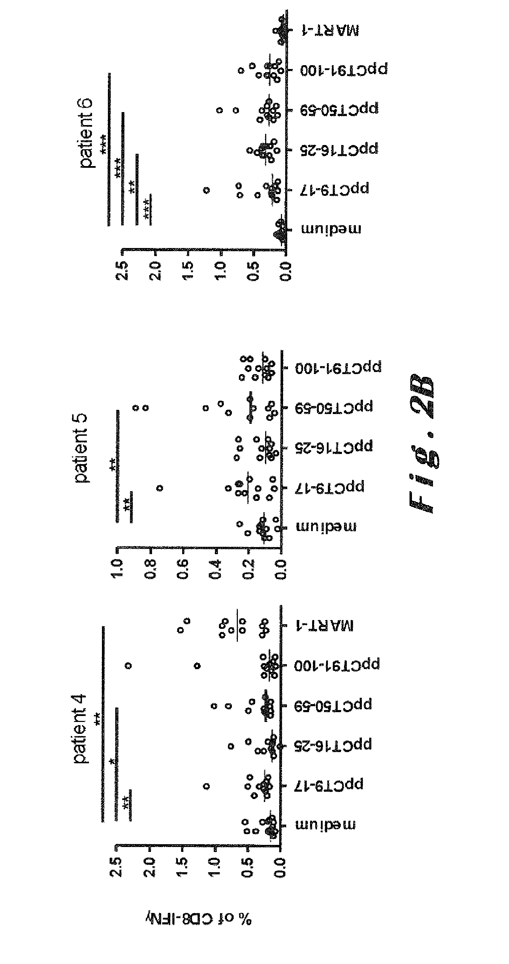

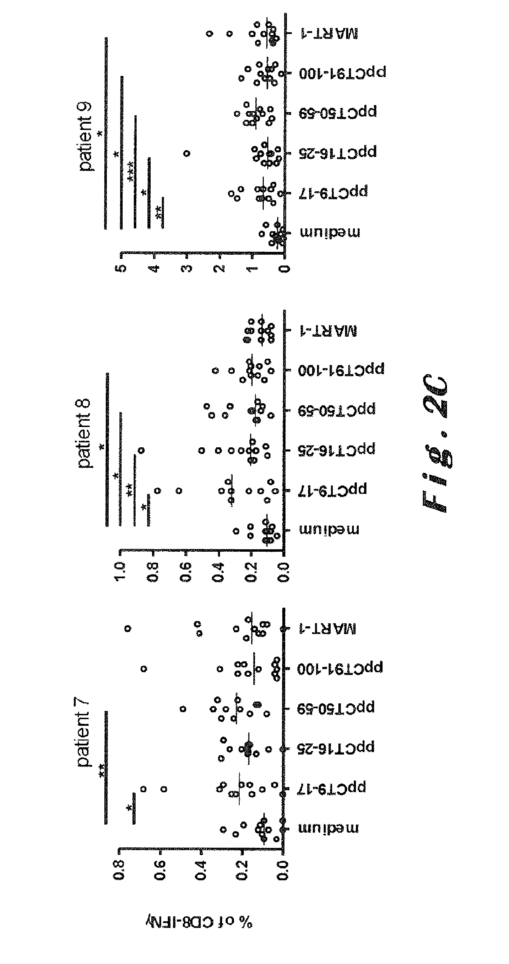

Figure 2 : Stimulation *in vitro* de PBMC de patients avec les peptides de SEQ ID NO : 4-7. Les PBMC des 9 patients atteints de cancer bronchique non à petites cellules (CBNPC) ont été stimulés avec chacun des 4 peptides puis le nombre de cellules CD8$^+$/IFN$\gamma^+$ a été analysé par cytométrie de flux. Chaque point (n) correspond à 8 puits indépendants de stimulation. La médiane de ces points est représentée pour chaque patient et chaque peptide (n=12). Les augmentations statistiquement significatives sont indiquées (*p<0.05 ; **p<0.01 ; ***p<0.005).

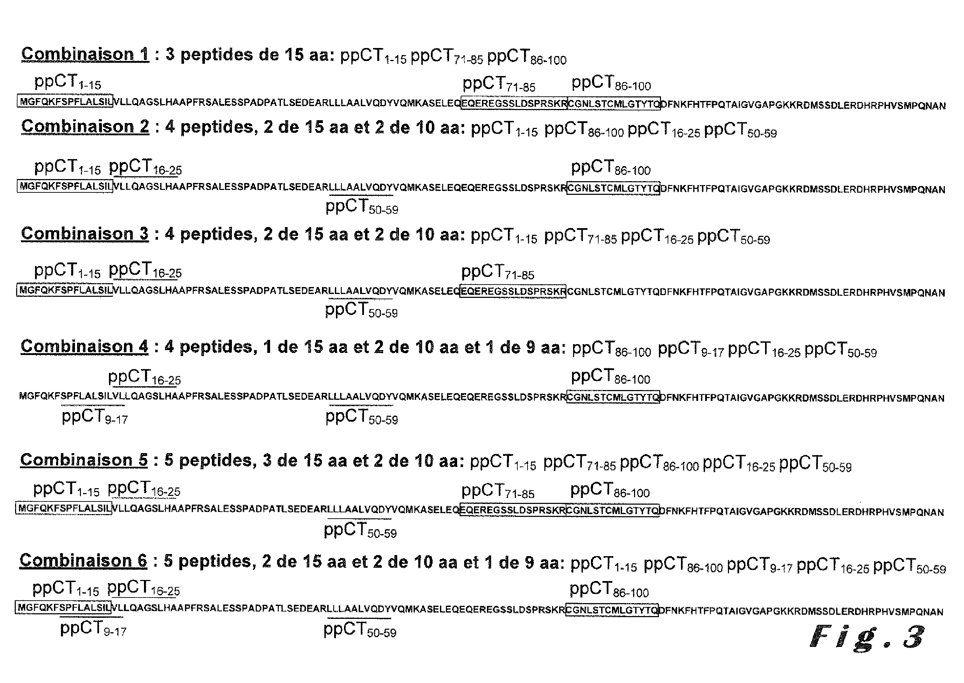

Figure 3 : Résumé des différentes combinaisons peptidiques testées *in vitro* et *in vivo* dans un modèle de souris transgéniques pour les molécules HLA-A2.

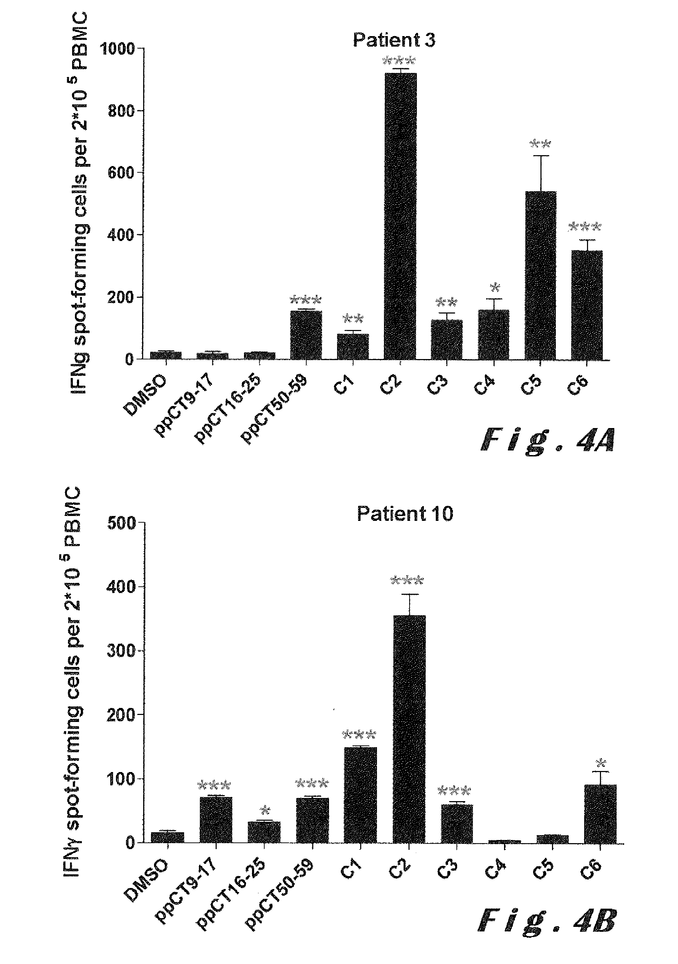

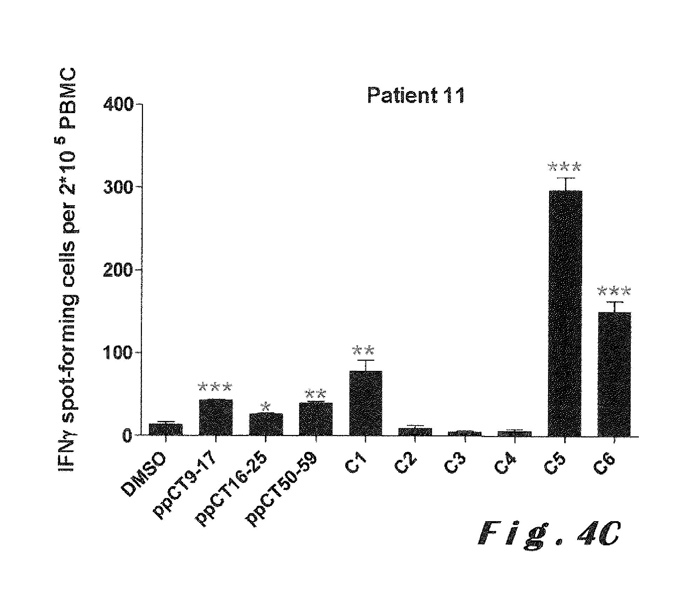

Figure 4 : Stimulation *in vitro* des PBMC de patients avec les peptides courts de 9-10 aa ou les différentes combinaisons peptidiques. Les PBMC des patients 3, 10 et 11 ont été stimulés soit avec un des peptides SEQ ID Nos : 4 à 6, seul, soit avec une des combinaisons 1 à 6 décrites en figure 3 ; le nombre de cellules sécrétant de l'IFN$\gamma^+$ a ensuite été analysé par ELISPOT. Les augmentations statistiquement significatives sont indiquées (*p<0.05 ; **p<0.01 ; ***p<0.005).

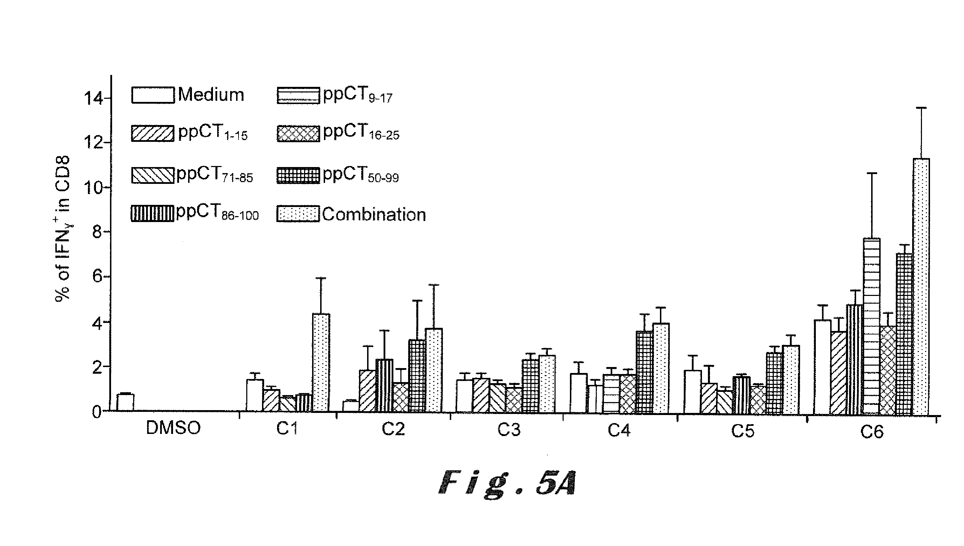

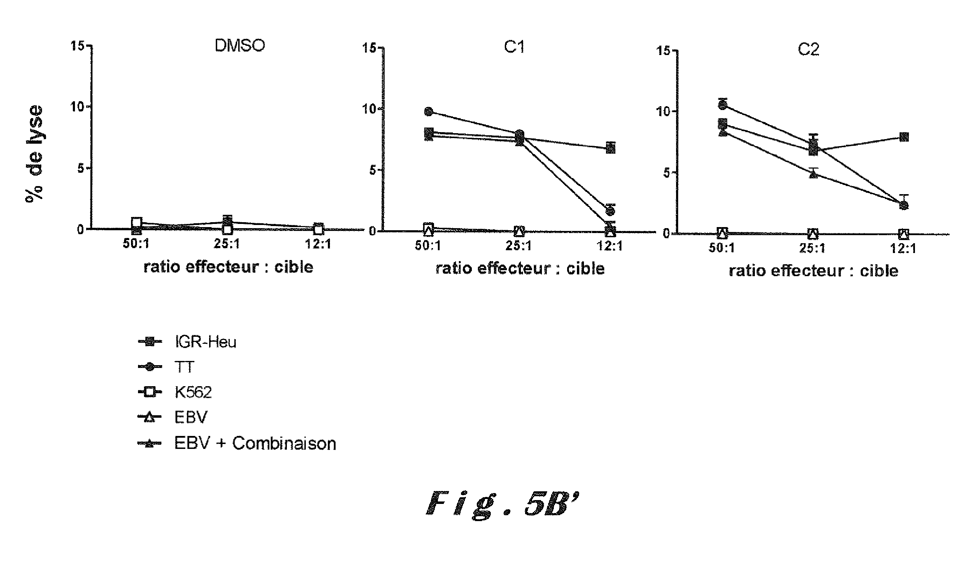

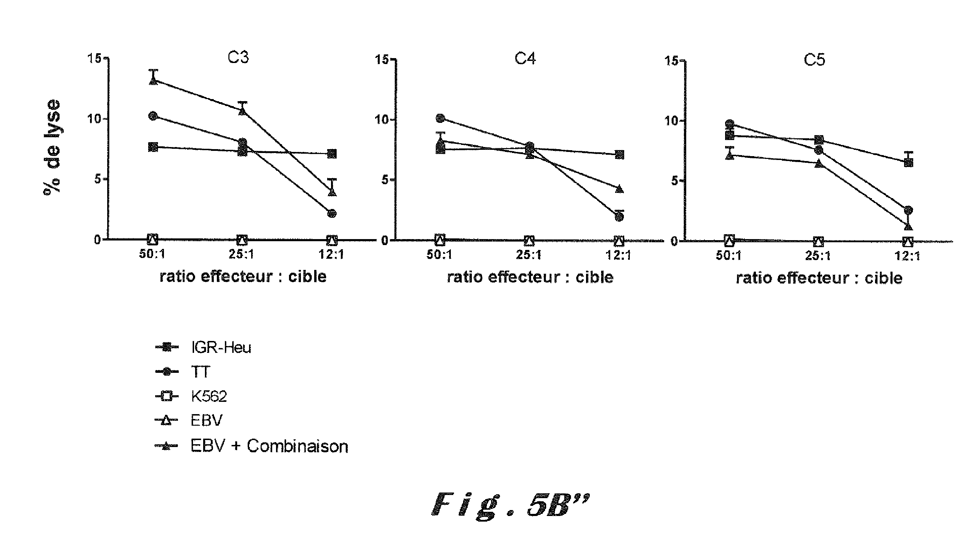

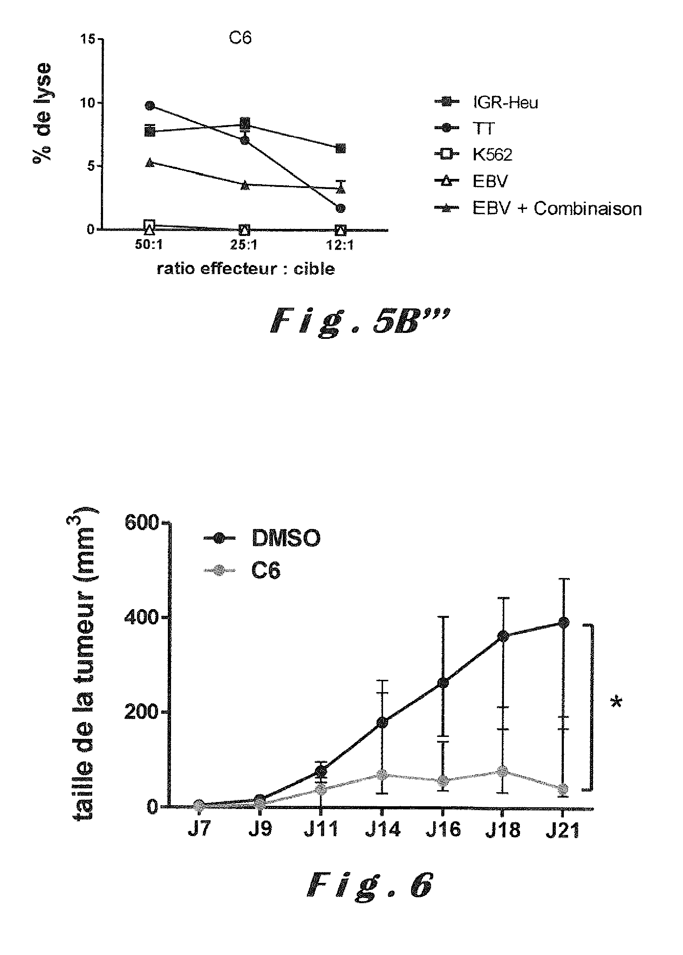

Figure 5 : Immunisation des souris transgéniques pour les molécules HLA-A2 avec les différentes combinaisons peptidiques **A.** Pourcentage de lymphocytes T CD8$^+$/IFN$\gamma^+$ après restimulation *ex vivo* des splénocytes avec chacun des peptides seul ou en combinaison (représentés par des colonnes de couleurs différentes). **B.** Réponse cytotoxique des lymphocytes T CD8 après immunisation des souris avec les différentes combinaisons peptidiques (C1-C6).

Figure 6 : Effet antitumoral de la combinaison peptidique C6 dans le modèle de souris transgénique pour les molécules HLA-A2. $1 \times 10^6$ cellules tumorales D122-ppCT ont été greffées en sous-cutané dans le flanc des souris transgénique à J0. Les souris ont ensuite été vaccinées en sous-cutané à J0, J3, J4 et J14, avec $100 \mu M$ de chaque peptide composant la combinaison C6 et de $25 \mu g$ d'adjuvant. La taille de la tumeur est définie par la formule longueur*largeur*profondeur. La différence de croissance tumorale statistiquement significative est indiquée (*p<0.05).

## EXEMPLE 1 : STIMULATION *IN VITRO* DES PBMC DE PATIENTS PAR PEPTIDES DE 9 OU 10 ACIDES AMINES DE LA PREPROCALCITONINE. MATERIEL ET METHODES

### Peptides

Les peptides suivants ont été choisis:

ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

### Cellules utilisées et stimulation

Les PBMC (*peripheral blood mononuclear cells*) de 11 patients atteints de cancer bronchique non à petites cellules (CBNPC) ont été isolés par Ficoll à partir du sang périphérique. Pour chaque peptide, des PBMC ont été stimulés 2 fois (J0 et J7) avec 20 $\mu M$ de peptide. Les PBMC ont été cultivés pendant 2 semaines en plaques 96 puits, à raison de 20 millions de cellules / plaque, en milieu complet (RPMI1640, 10% SAB, 1% sodium pyruvate, 0.1% pénicilline/streptavidine) en présence d'IL-2 (20 UI/ml) + IL-4 (10 ng/ml) + IL-7 (10 ng/ml).

### Analyse par cytométrie

Après 15 jours, les PBMC sont de nouveau stimulés avec 2,5 $\mu M$ du peptide utilisé lors de la première étape de stimulation, en présence de 100 $\mu g/ml$ de Brefeldin A, et incubés pendant 6h à 37°C, 5% $CO_2$. Les cellules sont ensuite incubées 20 min. avec un anticorps anti-CD8-APC, puis lavées en PBS, fixées avec PBS-formaldéhyde 2% et perméabilisées par BSA/saponine. Elles sont ensuite incubées avec un anticorps anti-IFN$\gamma$-PE. L'analyse est réalisée par cytométrie en flux. L'étude statistique est réalisée par le test de Mann-Whitney et les augmentations statistiquement significatives sont indiquées (*p<0.05 ; **p<0.01 ; ***p<0.005).

**RESULTATS**

Les résultats sont illustrés dans la Figure 2.

L'analyse par cytométrie en flux de la réponse à la stimulation avec les différents peptides de 9 ou 10 acides aminés a permis de montrer que la stimulation par les peptides ppCT9-17, ppCT16-25, ppCT50-59 et ppCT91-100 permet une augmentation de 1 à 4,5 fois le niveau basal des lymphocytes T CD8$^+$/INF$\gamma^+$ chez les patients testés. L'analyse de différents puits de stimulation a permis de montrer que cette augmentation de la réponse CD8 est statistiquement significative (*p<0.05 ; **p<0.01 ; ***p<0.005). Néanmoins, les différents patients ne répondent pas de la même manière à chacun des peptides. Les patients 1 ; 2 ; 6 ; 8 et 9 déclenchent une sécrétion d'IFNγ par les lymphocytes T CD8 après stimulation par l'ensemble des peptides testés, alors que les patients 4, 5 et 7 ne déclenchent une réponse lymphocytaire qu'après stimulation avec les peptides ppCT9-17 et ppCT50-59 tandis que le patient 3 déclenche une réponse avec les peptides ppCT50-59 et ppCT91-100.

En conclusion, il apparait que les peptides sont immunogènes selon les patients testés et permettent une réponse immunitaire *via* les lymphocytes T CD8. Vu la variabilité de la réponse aux différents peptides, une combinaison peptidique aurait plus de chance d'induire une réponse anti-ppCT chez la majorité des patients inclus.

**EXEMPLE 2 : STIMULATION *IN VITRO* DE PATIENTS AVEC LES PEPTIDES COURTS DE 9-10 AA OU LES DIFFERENTES COMBINAISONS PEPTIDIQUES MATERIEL ET METHODES**

**Peptides**

Les peptides suivants ont été choisis:

ppCT1-15 : MGFQKFSPFLALSIL (SEQ ID NO: 1) ;
ppCT71-85: EREGSSLDSPRSKRC (SEQ ID NO: 2) ;
ppCT86-100: GNLSTCMLGTYTQDF (SEQ ID NO: 3) ;
ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

**Cellules utilisées et stimulation**

Les PBMC (*peripheral blood mononuclear cells*) de 3 patients atteint de cancer du poumon non à petites cellules ont été isolés par Ficoll à partir du sang périphérique, puis stimulés 2 fois (J0 et J7) avec 20 μM de chacun des peptides ou avec les combinaisons équimolaire de 20 μM de chaque peptide décrites dans la figure 3. Les PBMC sont cultivés pendant 2 semaines en plaques 96 puits, à raison de 20 millions de cellules / plaque, en milieu complet (RPMI1640, 10% SAB, 1% sodium pyruvate, 0.1% pénicilline/streptavidine) en présence d'IL-2 (20 UI/ml) + IL-4 (10 ng/ml) + IL-7 (10 ng/ml).

**Analyse par ELISPOT**

Après 15 jours, les PBMC sont récupérés puis sont incubés sur des plaques ELISPOT à raison de 100 000 cellules par puits, en présence de milieu avec 2,5 μM de chaque peptide ou avec les combinaisons équimolaires de 2,5 μM de chaque peptide utilisé pour la stimulation. Les plaques sont incubées 15 à 18h à 37°C et 5% de $CO_2$. Les spots IFNγ sont révélés suivant les instructions du fabriquant (Gen-Probe Diaclone). Les spots formés par les cellules sécrétant de l'IFNγ sont comptés et analysés avec le C.T.L Immunospot system (Cellular Technology Ltd). L'étude statistique est réalisé par le test de Mann-Whitney et les augmentations statistiquement significatives sont indiquées (*p<0.05 ; **p<0.01 ; ***p<0.005)

**RESULTATS**

Les résultats sont illustrés dans la Figure 4.

L'analyse du nombre de cellules sécrétrices d'IFNγ suite à la stimulation des PBMC de 3 patients avec les combinaisons décrites en figure 3 a permis de montrer que ces différentes combinaisons permettent de stimuler et ainsi d'amplifier les lymphocytes spécifiques de ces peptides. En effet, une augmentation significative du nombre de cellules sécrétrices d'IFNγ est observée pour 3 combinaisons chez le patient 11, 4 combinaisons chez le patient 10 et les 6 combinaisons chez le patient 3.

De plus, les stimulations par les combinaisons C2, C5 et C6 augmentent également le nombre de cellules sécrétant de l'IFNγ par rapport aux stimulations par les peptides seuls. Ces réponses sont patient-dépendantes. En effet, la stimulation des PBMC du patient 3 par les combinaisons C2, C5 et C6 permettent d'augmenter le nombre de cellules sécrétant de l'IFNγ de 6 fois pour C2, 3 fois pour C5 et 2 fois pour C6, alors que pour le patient 10, seule la stimulation des PBMC avec la combinaison C2 augmente le nombre de cellules sécrétant de l'IFNγ de 3 fois et la stimulation des PBMC du patient 11 par les combinaisons C5 et C6 permettent d'augmenter le nombre de cellules sécrétant de l'IFNγ de 3 fois pour C5 et 2 fois pour C6.

Ce résultat démontre que la combinaison de plusieurs peptides issus de l'antigène ppCT permet une augmentation de la réponse immunitaire développée par les lymphocytes T CD8. De plus, ces résultats démontrent également l'intérêt de l'utilisation d'une combinaison peptidique par rapport aux peptides seuls pour déclencher une réponse immunitaire chez la majorité des patients.

**EXEMPLE 3: IMMUNISATION DE SOURIS TRANSGENIQUES AVEC LES DIFFERENTES COMBINAISONS PEPTIDIQUES MATERIEL ET METHODES**

**Peptides**

Les peptides suivants ont été choisis:

ppCT1-15 : MGFQKFSPFLALSIL (SEQ ID NO: 1) ;
ppCT71-85: EREGSSLDSPRSKRC (SEQ ID NO: 2) ;
ppCT86-100: GNLSTCMLGTYTQDF (SEQ ID NO: 3) ;
ppCT9-17 FLALSILVL (SEQ ID NO:4) ;
ppCT16-25 VLLQAGSLHA (SEQ ID NO:5).
ppCT50-59 LLAALVQDYV (SEQ ID NO:6) ;
ppCT91-100 CMLGTYTQDF (SEQ ID NO:7) ;

**Cellules utilisées**

Les lignées cellulaires tumorales humaines TT (issue d'une tumeur médullaire de la thyroïde) et IGR-Heu (isolée à partir de la tumeur CBNPC du patient Heu), exprimant l'antigène ppCT, sont utilisées comme cellules cibles. La lignée K562 (issue d'un patient atteint d'une leucémie chronique myéloïde) est utilisée comme contrôle négatif. La lignée Heu-EBV, (issue des lymphocytes B du patient Heu) est utilisée pour déterminer la spécificité de la lyse. Après marquage avec le $^{51}$Cr, les cellules Heu-EBV sont incubées avec le mélange équimolaire à 20 μM de chaque peptide (décrit dans la Figure 4).

**Immunisation des souris**

Les souris transgéniques pour les molécules HLA-A2 ont été immunisées 4 fois (J0, J7, J14 et J21) en sous-cutané au niveau du flanc avec 100 μl de combinaisons peptidiques contenant un mélange équimolaire de 100 μM de chacun des peptides et d'un adjuvant (25 μg de Poly (I:C)) (Figure 4). 28 jours après la première injection, les souris ont été sacrifiées puis les splénocytes ont été récupérés et cultivés une nuit en milieu complet (RPMI1640, 10% SVF, 1% sodium pyruvate, 0,1% pénicilline/streptavidine) en présence d'IL-2 (20 UI/ml).

**Analyse par cytométrie et analyses par test de cytotoxicité**

Le lendemain, les splénocytes ont été stimulés avec 2,5 μM de chacun des peptides, seul ou en combinaison, en présence de 10 μg/ml de Brefeldin A et incubés pendant 6 h à 37°C, 5% $CO_2$. Les cellules sont ensuite incubées 20 min avec un anticorps anti-CD8-PE, puis lavées en PBS, fixées avec PBS-formaldéhyde 2% et perméabilisées avec BSA/saponine. Elles sont ensuite incubées avec un anticorps anti-IFNγ-APC. L'analyse est réalisée par cytométrie en flux.

Pour l'analyse de l'activité cytotoxique, les lymphocytes T CD8 ont été isolés des splénocytes par déplétion négative (miltenyi biotech). L'activité cytotoxique des lymphocytes T CD8 ainsi obtenus a été évaluée par un test de relargage de chrome 51 ($^{51}$Cr). Les cellules cibles (décrites dans le paragraphe ci-dessus) sont incubées 1h à 37°C en présence de 20 μM de $^{51}$Cr. Les cellules sont ensuite lavées avec du RPMI avant d'être co-cultivées à 37°C avec les lymphocytes T CD8 à des rapports effecteur/cible de 50:1, 25:1 et 12:1. Après 4h d'incubation, la concentration de $^{51}$Cr relargué dans le surnageant des co-cultures est mesurée. Le pourcentage de lyse est calculé grâce à la formule suivante :

$$\% \, de \, lyse = \frac{(Chrome \, relagué - Chrome \, relagué \, minimun)}{(Chrome \, relagué \, maximun - Chrome \, relagué \, minimun)} X \, 100$$

**RESULTATS**

L'analyse par cytométrie en flux des splénocytes isolés suite à l'immunisation des souris avec les combinaisons peptidiques C1 à C6 montre une augmentation du pourcentage de lymphocytes T CD8 exprimant de l'IFNγ (Figure 5A). Cette augmentation est dépendante de la combinaison utilisée, allant d'un fold change de 3 à 13 (4,8 pour C1 ; 3 pour C2, 3,4 pour C3, 5,5 pour C4, 4,3 pour C5 et 13,6 pour C6). Ces résultats montrent que l'immunisation des souris transgéniques avec les différentes combinaisons peptidiques permet de développer des réponses immunitaires via l'induction de lymphocytes T CD8.

De plus, la stimulation *ex vivo* avec chacun des peptides issus de la combinaison permet de démontrer l'effet additionnel des combinaisons. Pour la combinaison C1, on observe que la réponse après stimulation *ex vivo* augmente d'un fold change de 4,2 ; 5,2 et 4,6 par rapport aux stimulations *ex vivo* par les peptides ppCT1-15 ; ppCT71-85 et ppCT86-100 respectivement. La même observation peut être faite sur l'efficacité de la réponse immunitaire via les lymphocytes T CD8 pour l'ensemble des combinaisons. Néanmoins, aucune des combinaisons ne montre d'effet sur le peptide ppCT50-59, dû notamment à la forte réponse qu'il induit naturellement.

Enfin, les lymphocytes T CD8 générés par les immunisations des souris avec les combinaisons peptidiques sont capables de reconnaitre et de lyser de façon spécifique les cellules exprimant l'antigène ppCT, comme IGR-Heu et TT (Figure 5B). En effet, la figure 5B montre que les lymphocytes T CD8 issus de splénocytes de souris non immunisées ne sont pas capables de lyser ni la lignée tumorale IGR-Heu ni la lignée tumorale TT, contrairement aux lymphocytes T CD8 issus de splénocytes de souris immunisées. Suivant les conditions d'immunisation, les lymphocytes lysent entre 7 et 15% des cellules exprimant l'antigène ppCT. Les lymphocytes T ne lysent pas les cellules K562 ou les cellules Heu-EBV sauf si celles-ci sont chargées avec la combinaison peptidique. Ce résultat montre la spécificité de la lyse obtenue pour les cellules tumorales exprimant l'antigène ppCT.

L'ensemble des résultats, obtenus par l'immunisation des souris transgéniques, démontre que l'utilisation de combinaisons peptidiques permet d'activer et d'augmenter la réponse immunitaire exercée par les lymphocytes T CD8 contre les cellules exprimant l'antigène ppCT.

**EXEMPLE 4: VACCINATION DES SOURIS TRANSGENIQUES AVEC LA COMBINAISON C6**

**MATERIEL ET METHODES**

**Peptides**

Les peptides suivants ont été choisis:

ppCT1-15 : MGFQKFSPFLALSIL (SEQ ID NO: 1) ;
ppCT86-100: GNLSTCMLGTYTQDF (SEQ ID NO: 3) ;
ppCT9-17 FLALSILVL (SEQ ID NO:4) ;
ppCT16-25 VLLQAGSLHA (SEQ ID NO:5) ;
ppCT50-59 LLAALVQDYV (SEQ ID NO:6).

**Cellules utilisées**

La lignée murine LL2 (Lewis lung carcinoma) transgénique pour les molécules HLA-A2 (lignée D122) a été infectée avec un lentivirus exprimant la GFP et qui code pour l'antigène humain ppCT. $10^6$ cellules ont ensuite été injectées en sous cutané au niveau du flanc des souris.

**Immunisation des souris**

Les souris transgéniques pour les molécules HLA-A2 ont été vaccinées 4 fois (J0, J4, J7 et J14) en sous-cutané au niveau du flanc avec 100 µl de la combinaison peptidique C6 contenant un mélange équimolaire de 100 µM de chacun des peptides et d'un adjuvant (25 µg de Poly (I:C)).

**Suivi de la croissance tumorale**

Après la greffe des cellules tumorales, le poids des souris a été suivi tous les jours, et les tumeurs ont été mesurées tous les 2-3 jours à partir du 7ème jour. La taille est calculée en $mm^3$ de la façon suivante : Longueur x largeur x profondeur. La différence de croissance tumorale statistiquement significative est calculée par un t test et indiquée (*$p < 0.05$).

**RESULTATS**

Dans le modèle de souris transgénique pour les molécules HLA-A2 ayant une tumeur exprimant la ppCT, la vaccination avec la combinaison C6 induit une réduction de la croissance tumorale de façon significative par rapport aux souris non vaccinées. En effet, au bout de 21 jours, seule une souris vaccinée sur 3 possède une tumeur d'une taille de 190 $mm^3$ alors que les 3 souris contrôles ont développé une tumeur avec une taille moyenne de 350$mm^3$ (Figure 6).

Ce résultat montre la capacité de la vaccination avec la combinaison C6 à générer une réponse immunitaire efficace contre les tumeurs exprimant la ppCT, démontrant ainsi l'intérêt de l'utilisation de ces combinaisons en immunothérapie anti-tumorale.

**Revendications**

1. Composition **caractérisée en ce qu'**elle comprend au moins deux séquences peptidiques choisies parmi les séquences SEQ ID Nos : 1 à 18, chacune desdites séquences étant présente sous forme d'un peptide de 8 à 15 acides aminés ou incluse dans un polypeptide multiépitopique chimérique.

2. Composition selon la revendication 1, **caractérisée en ce qu'**elle comprend un mélange de peptides de 8 à 15 acides aminés.

3. Composition selon la revendication 1 ou la revendication 2, **caractérisée en ce qu'**elle comprend un polypeptide chimérique comprenant au moins deux séquences choisies parmi les séquences SEQ ID Nos : 1 à 18, lesdites séquences étant adjacentes, liées par un élément de liaison constitué par 1 à 5 acides aminés, ou séparées par une séquence comprenant un épitope d'une protéine autre que la préprocalcitonine.

4. Composition selon l'une quelconque des revendications précédentes, **caractérisée en ce qu'**elle comprend au moins deux séquences peptidiques choisies parmi les séquences SEQ ID Nos : 1 à 7.

5. Composition selon l'une quelconque des revendications précédentes, **caractérisée en ce qu'**elle comprend au moins trois séquences peptidiques choisies parmi les séquences SEQ ID Nos : 1 à 7.

6. Composition selon l'une quelconque des revendications précédentes, **caractérisée en ce qu'**elle comprend au moins une séquence choisie parmi les séquences SEQ ID No : 4 et 5, et la séquence SEQ ID No : 6.

7. Composition selon l'une quelconque des revendications précédentes, **caractérisée en ce qu'**elle comprend une combinaison de séquences peptidiques choisi parmi la combinaison des séquences SEQ ID Nos : 1, 2, 3, 5 et 6, la combinaison des séquences SEQ ID Nos : 1, 3, 4, 5 et 6, la combinaison des séquences SEQ ID Nos : 1, 2, 5 et 6, la combinaison des séquences SEQ ID Nos : 1, 3, 5 et 6 et la combinaison des séquences SEQ ID Nos : 3, 4, 5 et 6.

8. Composition comprenant au moins une molécule d'acide nucléique codant pour au moins deux peptides de 8 à 15 acides aminés choisis parmi les séquences SEQ ID Nos : 1 à 18 ou codant pour un polypeptide chimérique tel que défini à la revendication 3.

9. Composition selon l'une quelconque des revendications 1 à 7, comprenant des cellules présentatrices de l'antigène chargées avec lesdites séquences peptidiques.

10. Composition selon l'une quelconque des revendications 1 à 9, pour utilisation comme médicament.

11. Composition selon l'une quelconque des revendications 1 à 9, pour utilisation en immunothérapie anti-tumorale.

12. Composition selon la revendication 11, pour son utilisation selon la revendication 10, **caractérisée en ce que** ladite composition est destinée à l'immunothérapie de tumeurs exprimant la calcitonine, ou de pathologies associées à un taux sérique élevé de calcitonine, de précalcitonine, ou de prépro-$\alpha$-CGRP.

13. Composition selon la revendication 11, pour son utilisation selon la revendication 11 ou la revendication 12, **carac-**

**térisée en ce que** ladite composition est destinée à l'immunothérapie d'un carcinome médullaire de la thyroïde ou d'un carcinome pulmonaire.

14. Composition selon la revendication 11, pour son utilisation selon l'une quelconque des revendications 11 à 13, **caractérisée en ce que** ladite composition est destinée à l'immunothérapie d'une tumeur dont les cellules n'expriment pas les transporteurs de peptides TAP.

15. Composition selon la revendication 10, pour son utilisation selon l'une quelconque des revendications 10 à 14, **caractérisée en ce que** ladite composition est destinée au traitement d'un patient HLA-A*0201.

Calcitonin/α-CGRP gène (X15943) chromosome 11

α-CGRP mRNA

α-CGRP open reading frame

Calcitonin mRNA

Cacitonin open reading frame

Peptide Signal    57 aa    Calcitonin    27 aa

/ppCT₁₋₁₅  ppCT₁₆₋₂₅     ppCT₇₁₋₈₅    ppCT₈₆₋₁₀₀

MGFQKFSPFLALSILVLLQAGSLHAAPFRSALESSPADPATLSEDEARLLLAALVQDYVQMKASELEQEQEREGSSLDSPRSKRCGNLSTCMLGTYTQDFNKFHTFPQTAIGVGAPGKKRDMSSDLERDHRPHVSMPQNAN

ppCT₉₋₁₇       ppCT₅₀₋₅₉       ppCT₉₁₋₁₀₀

*Fig. 1*

EP 3 115 058 A1

Fig. 2A

*Fig. 2B*

Fig.2C

**Combinaison 1** : 3 peptides de 15 aa: ppCT$_{1-15}$ ppCT$_{71-85}$ ppCT$_{86-100}$

ppCT$_{1-15}$                                                     ppCT$_{71-85}$        ppCT$_{86-100}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQ|DFNKFHTFPQTAIGVGAPGKKRDMSSDLERDHRPHVSMPQNAN

**Combinaison 2** : 4 peptides, 2 de 15 aa et 2 de 10 aa: ppCT$_{1-15}$ ppCT$_{86-100}$ ppCT$_{16-25}$ ppCT$_{50-59}$

ppCT$_{1-15}$ ppCT$_{16-25}$                                                            ppCT$_{86-100}$

MGFQKFSPFLALSIL|VLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN

                                            ppCT$_{50-59}$

**Combinaison 3** : 4 peptides, 2 de 15 aa et 2 de 10 aa: ppCT$_{1-15}$ ppCT$_{71-85}$ ppCT$_{16-25}$ ppCT$_{50-59}$

ppCT$_{1-15}$ ppCT$_{16-25}$                                           ppCT$_{71-85}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQDFNKFHTFPQTAIGVGAPGKKRDMSSDLERDHRPHVSMPQNAN

                                            ppCT$_{50-59}$

**Combinaison 4** : 4 peptides, 1 de 15 aa et 2 de 10 aa et 1 de 9 aa: ppCT$_{86-100}$ ppCT$_{9-17}$ ppCT$_{16-25}$ ppCT$_{50-59}$

        ppCT$_{16-25}$                                                   ppCT$_{86-100}$

MGFQKFSPFLALSILVLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN

    ppCT$_{9-17}$                        ppCT$_{50-59}$

**Combinaison 5** : 5 peptides, 3 de 15 aa et 2 de 10 aa: ppCT$_{1-15}$ ppCT$_{71-85}$ ppCT$_{86-100}$ ppCT$_{16-25}$ ppCT$_{50-59}$

ppCT$_{1-15}$ ppCT$_{16-25}$                                           ppCT$_{71-85}$       ppCT$_{86-100}$

MGFQKFSPFLALSIL|VLLQAGSLHAAPFRSALESSPADPATLSEDEARLLLAALVQDYVQMKASELEQ|EQEREGSSLDSPRSKR|CGNLSTCMLGTYTQ|DFNKFHTFPQTAIGVGAPGKKRDMSSDLERDHRPHVSMPQNAN

                                            ppCT$_{50-59}$

**Combinaison 6** : 5 peptides, 2 de 15 aa et 2 de 10 aa et 1 de 9 aa: ppCT$_{1-15}$ ppCT$_{86-100}$ ppCT$_{9-17}$ ppCT$_{16-25}$ ppCT$_{50-59}$

ppCT$_{1-15}$ ppCT$_{16-25}$                                                   ppCT$_{86-100}$

MGFQKFSPFLALSIL|VLLQAGSLHAAPFRSALESSPADPATLSEDEARLLLAALVQDYVQMKASELEQEQEREGSSLDSPRSKR|CGNLSTCMLGTYTQ|DFNKFHTFPQTAIGVGAPGKKRDMSSDLERDHRPHVSMPQNAN

    ppCT$_{9-17}$                        ppCT$_{50-59}$

*Fig. 3*

EP 3 115 058 A1

*Fig. 4A*

*Fig. 4B*

Fig. 4C

*Fig. 5A*

**DMSO**

**C1**

**C2**

% de lyse

ratio effecteur : cible

ratio effecteur : cible

ratio effecteur : cible

IGR-Heu
TT
K562
EBV
EBV + Combinaison

*Fig. 5B'*

**C3** **C4** **C5**

% de lyse

ratio effecteur : cible    ratio effecteur : cible    ratio effecteur : cible

- IGR-Heu
- TT
- K562
- EBV
- EBV + Combinaison

*Fig.5B"*

*Fig. 5B''''*

*Fig. 6*

Europäisches
Patentamt

European
Patent Office

Office européen
des brevets

**RAPPORT DE RECHERCHE EUROPEENNE**

Numéro de la demande

EP 15 17 6174

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | WO 01/75179 A2 (LUDWIG INST CANCER RES [US]; INST NAT SANTE RECH MED [FR]; ROUSSY INST) 11 octobre 2001 (2001-10-11) <br> * page 9, peptide 28-36 * <br> * page 11, peptides 9-17 et 16-25 * <br> * page 15, peptide 91-100 * <br> * page 20, dernier alinéa - page 21, dernier alinéa * <br> * revendication 14 * <br> ----- | 1-15 | INV. A61K39/00 |
| A | PAPEWALIS C ET AL: "Dendritic cell vaccination induces tumor epitope-specific Th1 immune response in medullary thyroid carcinoma", HORMONE AND METABOLIC RESEARCH, vol. 40, no. 2, février 2008 (2008-02), pages 108-116, XP8173300, ISSN: 0018-5043 <br> * page 113, colonne de droite, dernier alinéa - page 114, alinéa 1 * <br> * page 115 * <br> ----- | 1-15 | |

DOMAINES TECHNIQUES RECHERCHES (IPC)

A61K

Le présent rapport a été établi pour toutes les revendications

| Lieu de la recherche | Date d'achèvement de la recherche | Examinateur |
|---|---|---|
| La Haye | 28 janvier 2016 | Mata Vicente, Teresa |

CATEGORIE DES DOCUMENTS CITES

X : particulièrement pertinent à lui seul
Y : particulièrement pertinent en combinaison avec un
    autre document de la même catégorie
A : arrière-plan technologique
O : divulgation non-écrite
P : document intercalaire

T : théorie ou principe à la base de l'invention
E : document de brevet antérieur, mais publié à la
    date de dépôt ou après cette date
D : cité dans la demande
L : cité pour d'autres raisons
 
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 15 17 6174

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

28-01-2016

| Document brevet cité au rapport de recherche | | Date de publication | Membre(s) de la famille de brevet(s) | | Date de publication |
|---|---|---|---|---|---|
| WO 0175179 | A2 | 11-10-2001 | AU | 5385201 A | 15-10-2001 |
| | | | EP | 1301624 A2 | 16-04-2003 |
| | | | WO | 0175179 A2 | 11-10-2001 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2009010874 A
- WO 1999003499 A

**Littérature non-brevet citée dans la description**

- **MORRIS et al.** *Nature,* 1984, vol. 308, 746-8
- **ROSENFELD et al.** *Nature,* 1983, vol. 304, 129-35
- **ZAIDI et al.** *Crit Rev Clin Lab Sci,* 1990, vol. 28, 109-74
- **COOMBES et al.** *Lancet,* 1974, vol. 1, 1080-3
- **MILHAUD et al.** *Lancet,* 1974, vol. 1, 462-3
- **SCHOTT et al.** *Cancer Immunol Immunother,* 2002, vol. 51, 663-8
- **DADAGLIO et al.** *Int. Immunol,* 2003, vol. 15, 1423-1430
- **VIAUD et al.** *J Immunother,* 2011, vol. 34, 65-75
- **TABOLLI et al.** *Tumori,* 1983, vol. 69, 227-230
- **SIM et al.** *Ann Clin Lab Sci.,* 1996, vol. 26, 487-95
- **CONTE et al.** *Acta Endocrinol,* 1984, vol. 106, 109-11
- **TAKUBO et al.** *Haematologia,* 2001, vol. 31, 177-9
- **KIEFER et al.** *Leuk Lymphoma,* 1994, vol. 13, 501-507
- **GHILLIANI et al.** *Cancer Res,* 1989, vol. 49, 6845-6851
- **KIEFER et al.** *Leuk Lymphoma.,* 1994, vol. 13, 501-507